# EUROPEAN PATENT APPLICATION

(11) **EP 4 190 296 A1**
(43) Date of publication of application: **07.06.2023**
(21) Application number: 21866498.5
(22) Date of filing: 20.08.2021
(51) Int. Cl.: A61F 13/84

(54) **WEARABLE DUMMY FOR TESTING BODY LIQUID ABSORBENT ARTICLE, AND TEST METHOD FOR USING SAME**

(30) Priority: 09.09.2020 JP 2020151575
(71) Applicant: Daio Paper Corporation, Ehime 799-0492 (JP)
(72) Inventor: RI, Mizuki, Shikokuchuo-shi, Ehime 799-0113 (JP)
(74) Representative: Williams Powell
(86) International application number: PCT/JP2021/030567
(87) International publication number: WO 2022/054538

(57) **Abstract**

A fitting dummy for testing on a body-fluid-absorbent article is disclosed, which, with an absorbent article fit on the dummy, allows clear capture of absorption, diffusion, or the like, of body fluid. The dummy 10 has the external shape representing the external shape of a human body including at least the torso and a pair of thighs, the dummy 10 is hollow in at least the torso and the thighs, the wall thickness t between the exterior surface and the corresponding interior surface of the dummy 10 is 3 to 15 mm except for a body fluid discharge area, the body fluid discharge area is provided with through holes 12a, 14a communicating with the hollow and the outside, and the dummy 10 is a transparent resin molded body.

## Description

### FIELD OF ART

The present invention relates to fitting dummies for body fluid absorption tests on body-fluid-absorbent articles, such as disposable diapers, sanitary napkins, and absorbent pads.

### BACKGROUND ART

Body-fluid-absorbent articles, such as disposable diapers, sanitary napkins, and absorbent pads, are often subjected to tests for the purpose of deciding their specifications, researching their performance, or the like, by fitting an article on a so-called dummy composed of a torso and a pair of thighs, supplying simulated body fluid through the dummy, and visually observing the absorption, diffusion, or the like, of the body fluid in the body-fluid-absorbent articles.

Conventionally, such a dummy is known to be produced by casting the overall torso and thighs with silicon rubber. That is, the overall torso and thighs of the dummy are solid inside.

Combination of the facts that the conventional dummy is solid inside and thus heavy, is made of silicon rubber, and has a higher optical refraction due to its internal solidity, has been posing difficulties to visual observation of absorption and diffusion of body fluid through the dummy.

Patent Publication 1 discloses a proposal for reproduction of the state of an absorbent article fit on a human body. This, however, does not allow monitoring observation of actual absorption and diffusion of body fluid.

### PRIOR ART PUBLICATION

### PATENT PUBLICATION

Patent Publication 1: JP 2018-183491 A

### SUMMARY OF THE INVENTION

### PROBLEMS TO BE SOLVED BY THE INVENTION

Therefore, the present invention resides in providing a fitting dummy for testing on a body-fluid-absorbent article which, with an absorbent article fit thereon, allows clear capture of absorption, diffusion, or the like of body fluid.

### MEANS FOR SOLVING THE PROBLEM

The fitting dummy for testing on a body-fluid-absorbent article according to the present invention solving the above-mentioned problem is a dummy having an external shape representing an external shape of a human body including at least a torso and a pair of thighs,
wherein the dummy is hollow in at least the torso and the thighs,
wherein a wall thickness between an exterior surface and a corresponding interior surface of the dummy is 3 to 15 mm except for a body fluid discharge area,
wherein the body fluid discharge area is provided with a through hole communicating with the hollow and outside, and
wherein the dummy is a transparent resin molded body.

Also proposed is a testing method using the fitting dummy for testing on a body-fluid-absorbent article, the method including:
fitting a body-fluid-absorbent article on the dummy,
supplying a simulated body fluid from inside of the hollow, and
capturing at least one phenomenon of absorption and diffusion, in the body-fluid-absorbent article, of the simulated body fluid transferred through the through hole into the body-fluid-absorbent article.

### EFFECT OF THE INVENTION

According to the present invention, with an absorbent article fit on the dummy, clear capturing of absorption, diffusion, or the like of body fluid may be conducted.

### BRIEF DESCRIPTION OF THE DRAWINGS

Fig. 1 is a schematic vertical sectional view of a dummy.
Fig. 2 is a perspective view (photograph) of a dummy in an upright position, seen from the torso end side.
Fig. 3 is a front view (photograph) of the dummy in the upright position.
Fig. 4 is a plan view (photograph) of a dummy in a sitting position, seen from the torso end side.
Fig. 5 is a front view (photograph) of the dummy in the sitting position.
Fig. 6 is a schematic vertical sectional view of a dummy illustrating the state of testing.

### EMBODIMENTS FOR CARRYING OUT THE INVENTION

The present invention will now be explained further with reference to the embodiments of the invention.

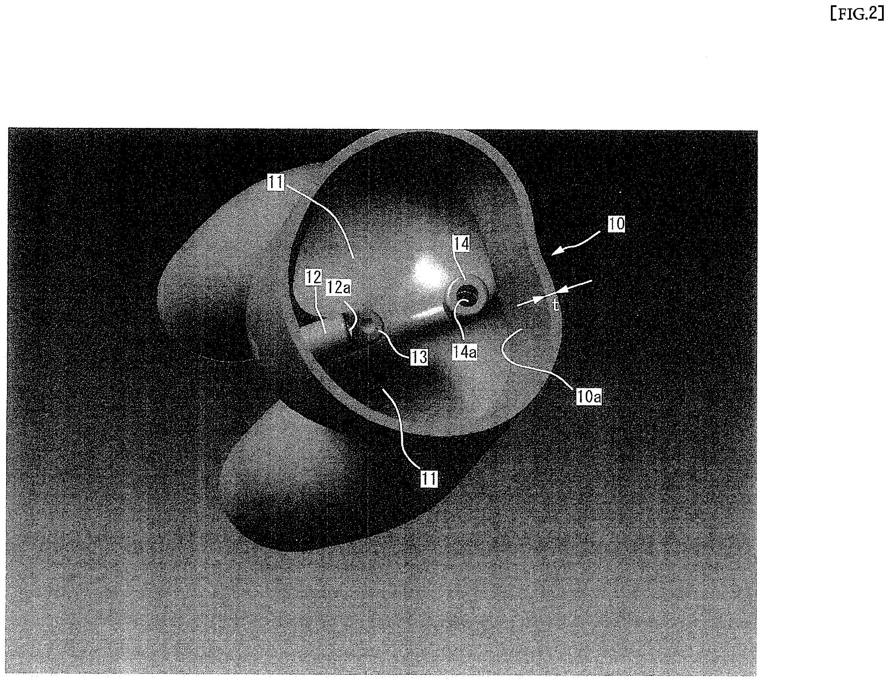

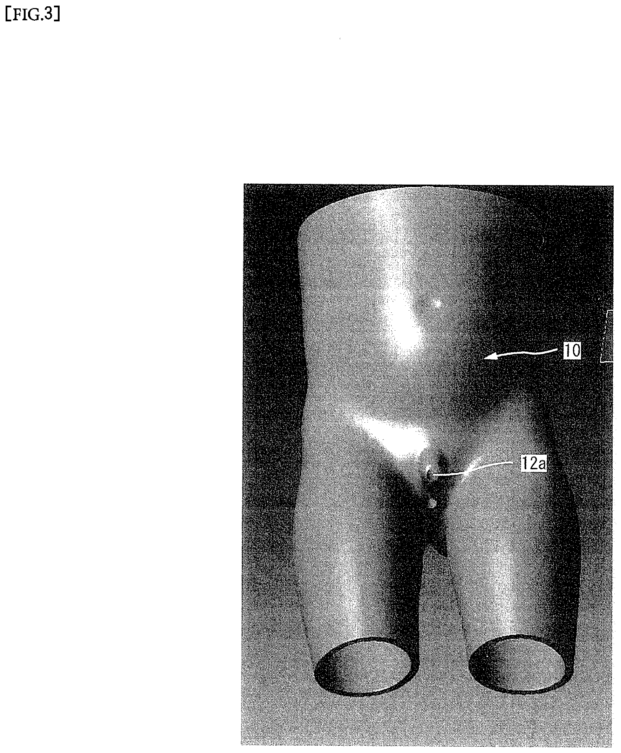

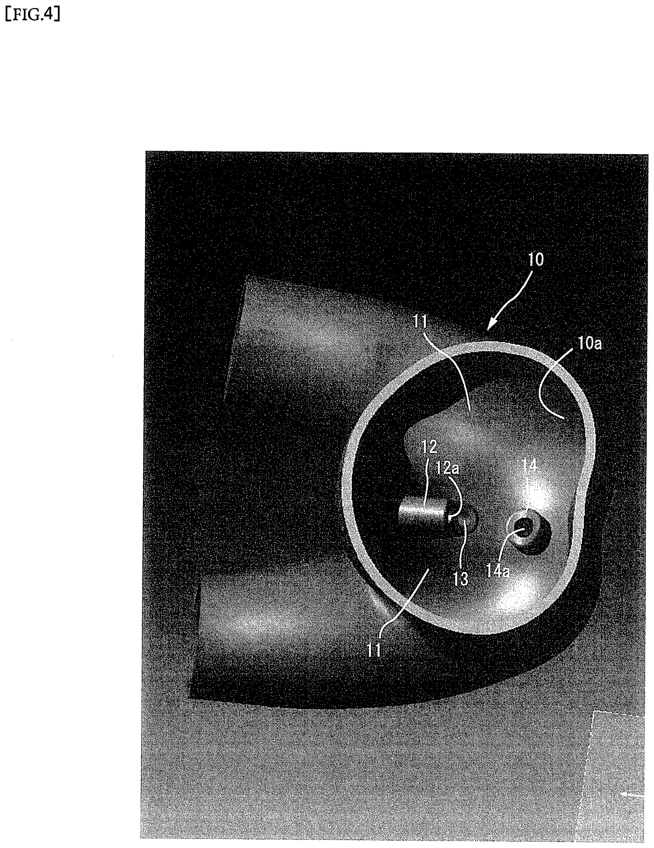

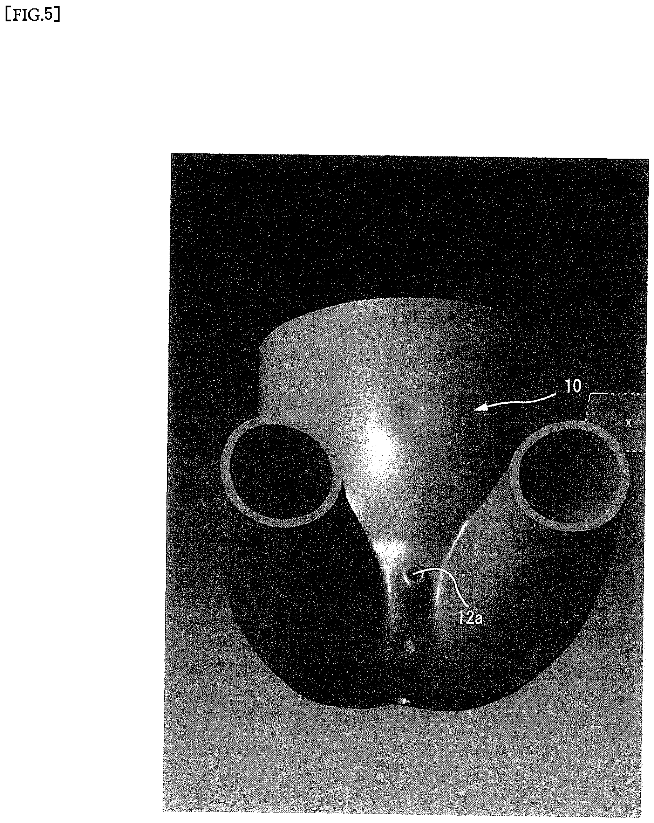

A dummy according to an embodiment of the invention is transparent and thus its appearance is hard to be indicated. In this regard, colored molded-body dummies are produced and photographed to show the appearance of the dummies in Figs. 2 to 5.

A dummy 10 according to an embodiment of the invention has an external shape representing the external shape of a human body including at least the torso and a pair of thighs.

The dummy may include the chest or the lower extremity, which are not essentially necessary, though, as an absorbent article is fit on the torso and the thighs.

The dummy 10 is hollow in the torso and the thighs. That is, the dummy 10 has a hollow 11 communicating with the torso end and the thighs.

The wall thickness t between the exterior surface and the corresponding interior surface 10a of the dummy 10 is 3 to 15 mm, except for body fluid discharge areas.

With a wall thickness of less than 3 mm, the dummy is vulnerable to damage, whereas with a wall thickness of over 15 mm, the dummy may be too heavy, and may cause optical refraction at the wall of the molded body to impair viewability therethrough.

The wall thickness t is more preferably 3 to 8 mm.

The body fluid discharge areas, i.e., a urea discharge area 12 and a loose stool discharge area 14, are provided with through holes 12a, 14a, respectively, communicating with the hollow 11 and the outside.

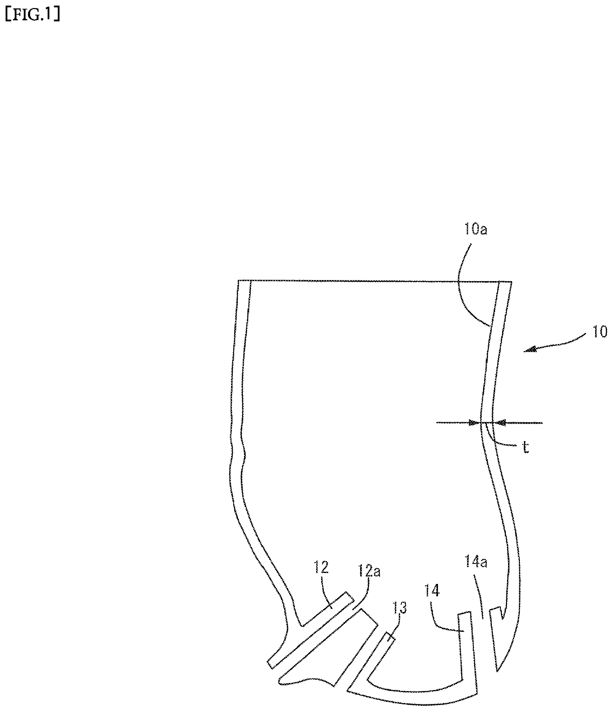

The reference numeral 13 refers to a menstrual blood discharge area for a female model, which is not provided with a through hole in an embodiment. This discharge area may be bored to form a through hole as necessary. Fig. 1 illustrates the state where the through hole is provided.

A higher viewability through the dummy is more preferred and, accordingly, the dummy is a transparent resin molded body.

The resin molded body may preferably be a molded body of an ultraviolet curable resin for ready molding.

The ultraviolet curable resin is a resin which forms a cross-linked structure through reaction of polymerizable oligomers or monomers having vinyl, acryloyl, epoxy, or the like groups, with radicals or cations generated by light (ultraviolet ray) irradiation being the initiating species.

Ultraviolet curable resins are categorized into a radical polymerization type and a cationic polymerization type, depending on the reaction mechanism (initiating species). Acrylate-based monomers or oligomers, which are of the radical polymerization type, may preferably be used. Ultraviolet absorption causes the initiator to generate radicals, which causes acrylate resin or acrylate monomers having acryloyl groups in the molecules thereof to undergo radical polymerization.

Photocationic polymerization may alternatively be employed, wherein ultraviolet absorption causes the initiator to generate acid, which causes vinyl monomers or cyclic ethers to initiate cationic polymerization.

Components for producing the ultraviolet curable resin are mainly polymerizable oligomers (prepolymers), polyfunctional monomers for controlling curability or physical properties, monofunctional monomers as a reactive diluent, and a photopolymerization initiator, and other additive components may optionally be used.

Examples of these components are as follows. For photoradical polymerization, the photopolymerizable oligomers may be urethane acrylate, epoxy acrylate, acryl acrylate, or polyester acrylate.

The photopolymerizable monomers may be polyfunctional acrylate monomers or monofunctional acrylate monomers.

The photopolymerization initiator may be benzophenone-, acetophenone-, benzoin ether-, or thioxanthone-based.

On the other hand, for photocationic polymerization, the photopolymerizable oligomers may be alicyclic epoxy, glycidyl-type epoxy, or oxetane compounds (cyclic ethers).

The photopolymerizable monomers may be vinyl ether monomers.

The photopolymerization initiator may be sulfonium salt- or iodonium salt-based.

The additives may include, in either case, polymerization inhibitors, fillers, pigments, dyes, antifoaming agents, or viscosity modifiers, but preferably fillers and pigments are not used.

In an embodiment, the body fluid discharge areas may preferably be provided with protrusions 12, 13, 14 protruding into the hollow 11, and through the protrusions 12, 13, 14 are preferably formed through holes 12a, (13a), 14a, respectively.

For testing, a body fluid discharge port need to be formed, but if a discharge port is formed through a wall portion with the wall thickness t without the protrusions 12, 13, 14 formed, cracks may be likely to occur, starting from the opening edges of the discharge port. In particular, the risk of cracks may be higher with the wall thickness t of 3 to 8 mm.

In contrast, with the protrusions 12, 13, 14 protruding into the hollow 11, so to speak reinforced areas are formed around the through holes 12a, (13a), 14a, which eliminate the risk of cracks. The length of the protrusions is preferably 5 to 30 mm. Excessive protrusion length impairs the viewability.

Note that the protrusions may have external protrusions on the other side of the wall but, most suitably, without such external protrusions, which could be an obstacle to body fluid diffusion. The length of the external protrusions, if any, is preferably short.

For representing a protruding male genital, the length of the external protrusion is preferably 8 to 20 mm for child dummies for conformity with the actual human body.

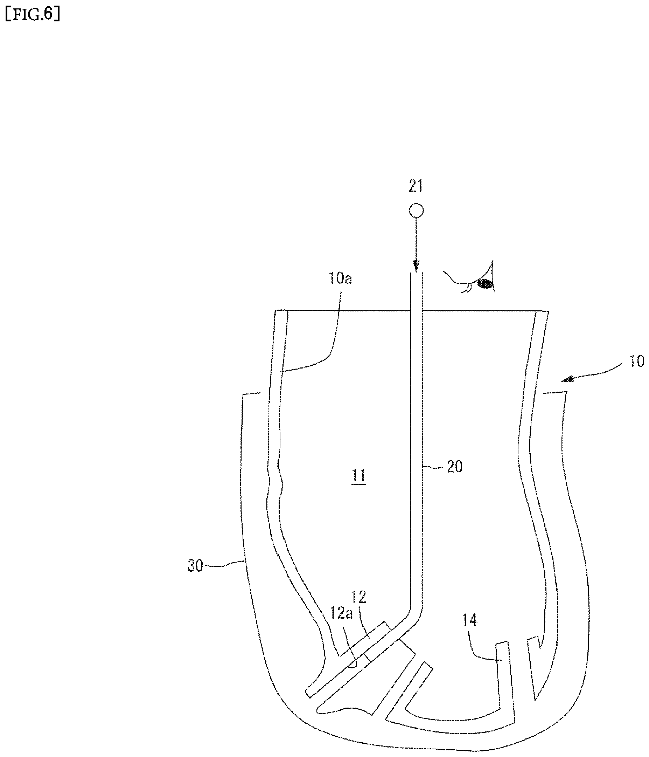

In testing, a body-fluid-absorbent article 30 under test is fit on the dummy 10 as shown in Fig. 6, simulated body fluid 21, such as artificial urine, simulated loose stool, or artificial menstrual blood, is supplied through the dummy 10, and the body fluid absorption and diffusion in the body-fluid-absorbent article is determined.

For example, as shown in Fig. 6, a transparent tube 20 may be inserted into the through hole 12a to supply the simulated body fluid 21.

In this case, in addition to visual observation by the tester, imaging with video or the like may be performed, and diffusion of body fluid over time may be monitored.

Further, distribution in amount of the body fluid in diffusion may be analyzed with images taken with a thermocamera or video.

### INDUSTRIAL APPLICABILITY

The present invention is applicable to tests on body-fluid-absorbent articles, such as disposable diapers, sanitary napkins, and absorbent pads.

### DESCRIPTION OF REFERENCE NUMERALS

10: dummy
11: hollow
12, 13, 14: protrusion (discharge area)
12a, 14a: through hole
20: tube
21: simulated body fluid
30: body-fluid-absorbent article

## Claims

1. A fitting dummy for testing on a body-fluid-absorbent article, the dummy having an external shape representing an external shape of a human body including at least a torso and a pair of thighs,
wherein the dummy is hollow in at least the torso and the thighs,
wherein a wall thickness between an exterior surface and a corresponding interior surface of the dummy is 3 to 15 mm except for a body fluid discharge area,
wherein the body fluid discharge area is provided with a through hole communicating with the hollow and outside, and
wherein the dummy is a transparent resin molded body.

2. The fitting dummy for testing on a body-fluid-absorbent article according to claim 1, wherein the resin molded body is a molded body of an ultraviolet curable resin.

3. The fitting dummy for testing on a body-fluid-absorbent article according to claim 1, wherein the body fluid discharge area is provided with a protrusion protruding into the hollow, and through the protrusion a through hole is formed.

4. The fitting dummy for testing on a body-fluid-absorbent article according to claim 1, wherein the wall thickness is 3 to 8 mm.

5. The fitting dummy for testing on a body-fluid-absorbent article according to claim 1, wherein the through hole is configured to receive a transparent tube therein for supply of a simulated body fluid.

6. A testing method using the fitting dummy for testing on a body-fluid-absorbent article according to any one of claims 1 to 5, the method comprising:
fitting a body-fluid-absorbent article on the dummy,
supplying a simulated body fluid from inside of the hollow, and
capturing at least one phenomenon of absorption and diffusion, in the body-fluid-absorbent article, of the simulated body fluid transferred through the through hole into the body-fluid-absorbent article.
